# EUROPEAN PATENT SPECIFICATION

(11) **EP 1 164 918 B1**
(45) Date of publication and mention of the grant of the patent: **03.12.2003**
(21) Application number: 00912885.1
(22) Date of filing: 29.03.2000
(51) Int. Cl.: A61B 3/00, A61B 3/117

(54) **A DEVICE FOR SELF EXAMINATION OF THE TRANSPARENT OPTICAL SYSTEM OF THE EYE AND A METHOD FOR USE THEREOF**
VORRICHTUNG ZUR SELBSTUNTERSUCHUNG DES TRANSPARENTEN OPTISCHEN SYSTEMS DES AUGES UND VERFAHREN ZUR VERWENDUNG DAZU
APPAREIL D'AUTO-EXAMEN DU SYSTEME OPTIQUE TRANSPARENT DE L'OEIL ET SON PROCEDE D'UTILISATION

(30) Priority: 29.03.1999 IL 12922699
(43) Date of publication of application: 02.01.2002
(73) Proprietor: Talia Technology Ltd., D.N. Harei Yehuda 90850 (IL)
(72) Inventor: ROZENMAN, Yaacov, 93714 Jerusalem (IL)
(74) Representative: Söllner, Udo, Dipl.-Ing.
(86) International application number: IL0000194
(87) International publication number: WO00057771

(56) References cited:
- US-A- 3 787 112
- US-A- 3 903 870
- US-A- 4 682 867
- US-A- 4 902 124
- US-A- 4 953 970

## Description

### FIELD OF THE INVENTION

The present invention relates to a device and a method for self examination of vision disorders involving opacities in the cornea, the lens and the vitreous gel of the eye.

### BACKGROUND OF THE INVENTION

Vision disorders are very often associated with opacities in the transparent optical system of the eye. Such opacities block the passage of light to the retina and cause vision disturbances. The most common disturbances of this type are caused by small opacities in the vitreous gel of the eye but these disturbances are usually only a passing inconvenience. A much more serious problem is opacities in the cornea or in the lens and in particular a cataract which is an opaque condition of the lens. Cataract might develop in the lens as a result of mechanical injury, metabolic deficiencies, or as result of advancing age (senile cataract) which is the most common form of cataract.

Most of the cataracts are painless and unaccompanied by inflammation. Traumatic cataract results from a direct or an indirect trauma to the eye. Senile cataract usually occurs in persons over 55 and generally involves both eyes. Starting in the form of streaks, dots or sheets of opacities, it develops gradually over several months or years and eventually makes the entire lens opaque. There is no single, valid objective test that indicates the presence of an operable cataract. Ophthalmologists analyze patient symptoms, perform a regular eye exam and may test for glare disability and contrast sensitivity to assist diagnosis. The final decision to proceed with the surgery lies with the patient. Cataract surgery is most often performed under local anesthesia. The clouded lens is either removed intact through a 6 -12 mm incision or is dissolved by a high frequency ultrasound (phaco emulsification). This second method, which requires a much smaller incision, approximately 3 mm, becomes the more favorable method for cataract removing. However, this method has best results when the cataract is not too mature. In a very mature cataract the dissolving of the hardened lens might require more energy and efforts.

It is therefore desirable that any person and especially persons who are over 50, will examine the status of the transparent optical system of their eyes on a routine basis. Such self examination is also desired by many persons who are aware of cataract. In special cases, when a beginning of cataract already had been diagnosed by an ophthalmologist, it is sometimes desirable that the patient will check himself between visits to his ophthalmologist, in order to follow up the cataract progress.

Various attempts were made to develop apparatuses and methods for self-examination of certain conditions of the eye.

U.S. patent No. 3,903,870 discloses methods and apparatuses for performing self-examination of the eye, the pattern thereby detected serving to warn the user to consult qualified professional advice if abnormalities are noted. The method comprises positioning an illuminated optical fiber of 10 mm diameter, or less, at the anterior focus of the eye, thereby to cause the eye to focus a parallel beam of light on the retina of the eye, the uniform retinal illumination caused by said point source permitting the user to see an image on his own retina.

U.S. patent No. 3,787112 discloses apparatus and method for self-examination of certain conditions of the eye, for example, the presence, location and nature of a cataract, retinal damage, scars and injuries, etc. Diffused light is introduced into the eye through an eyepiece supported coaxially of the pupilary axis thereby permitting the user to perceive a cataract on his eye and to view in very substantially enlarged form the cataract and/or certain conditions of his own eye on a translucent target or screen forwardly of his eye which target is preferably imprinted with a network of lines useful in the accurate transfer and recording of observed conditions and faults onto a sheet bearing a similar network.

U.S. patent No. 4,682,867 teaches an apparatus for self-examination of the human eye comprising a pen light for generating a beam of light, a reflecting sphere for orthogonally reflecting the beam through an opaque disc having a pin hole and a cylindrical head enclosing the reflecting sphere and opaque disc.

The reflecting sphere is adjustable along its axis, and functions to reflect a uniformly diffuse beam of light through the pin hole. When the pin hole is at the anterior focus of the human eye and along its pupilary axis, a viewer can see opacities in the eye.

The present invention provides a simple method and device for cataract self examination.

It is the aim of the present invention to provide a facile method and a facile device, for self examination of the transparent parts of the optical system of the eye.

### SUMMARY OF THE INVENTION

The present invention relates to a device for self examination of the transparent optical system of the eye comprising; a) a rigid hollow frame having one face with means for fitting said frame to the orbit of the eye; b) a small source of light located inside said frame c) means for adjusting the distance between the small source of light and cornea such that when said frame is fitted to eye, said distance is in the range 5 to 30, preferably 12 to 18 mm.

The small light source can be a primary light source such as a light emitting diode (LED) or can be a "virtual" light source, i.e., an element (or combination of elements) that transmit, reflects or refracts light originating from another primary light source. The primary light source can be part of the device or can be an outside available light source such as a lamp or the sun.

The rigid frame can be of any shape that allows a simple fitting of the device to the eye orbit, or which has means for simple and convenient positioning of the device at the right distance in front of the eye. The most commonly used means for positioning the device before the eye is an elastic rubber eyeguard as used in telescopes etc.

The position of the light source within the rigid frame is designed such that the distance between the light source and the eye, during eye examination, is in the range of the focal length of an average eye. Finer adjustment of the light source position in order to bring it to a distance *d* equal to the focal length of the examined eye, is done either by squeezing the elastic rubber eye guard against the eye orbit until a clear and defined full circle of light is viewed, or in a device which has means for adjusting the position of the light source within the rigid frame, by using these means.

The device according to the present invention further comprises means for clearing the beam of light falling on the cornea from disturbances resulting from dust and dirt on the optical elements of the device or in the light passage from light source to eye or from the phenomenon of speckles in the light source itself. These means can be motorized or manual means for fast rotation of the light source or elements in the path of the light such that the disturbances are averaged out, or a spatial filter comprising of a pinhole disk or a combination of a lens or a filter or a diffuser and a pinhole disk.

The device according to the present invention can further comprise a filter between the source of light and the edge of the device facing the eye, in order to select a specific range of the visible spectrum.

The present invention further relates to a method for self examination of opacities within the transparent optical system of the eye (cornea, lens and vitreous humor) comprising; a) aligning a small source of light in front of the eye at a distance of about 15 mm from the eye, wherein said source of light emits diverging rays toward the eye; b) fine adjusting of the distance between the light source and the eye until a clear full bright circle is observed by the examined eye; c) examining said circle for shadows wherein any shadow observed in the circle might indicate an opacity in the optical system of the eye.

The method according to the present invention further comprises rotating or moving the source of light in order to distinguish between shadows resulting from opacities within the eye to shadows resulting from inhomogeneities (resulting from dust and dirt or speckles) of the light beam, i.e, inhomogeneities in the optical system of the device.

### DESCRIPTION OF THE DRAWINGS:

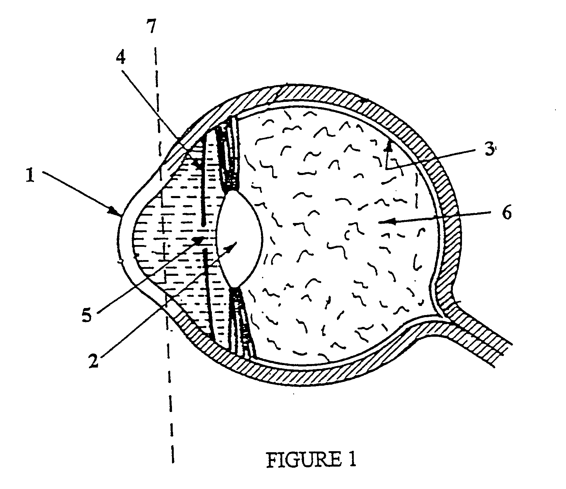

**Fig 1**: A schematic description of a human eye. **1** cornea; **2** lens; **3** retina **4** iris; **5** pupil; **6** vitreous gel ; **7** the first principal plane of the eye.

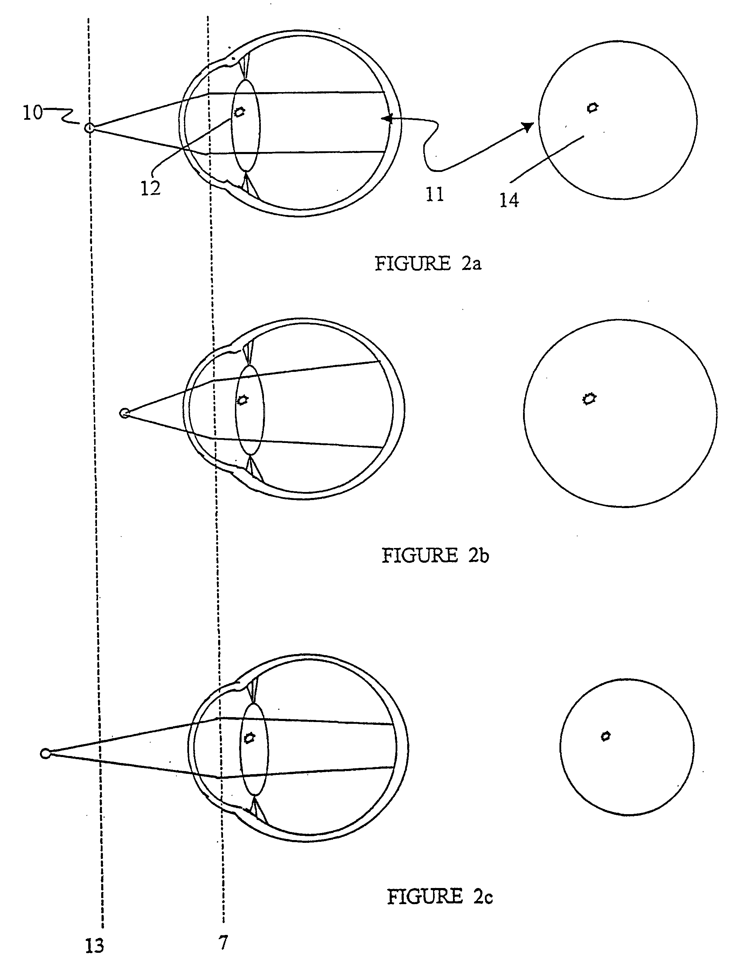

**Fig 2a**: diverging light rays emitted from a small light source **10** that is located at the anterior focal plane of a person's eye are bent at the cornea and at the lens (shown schematically in the drawing to bend at the first principal plane of the eye instead of bending twice at the cornea and twice at the lens **7**) and fall on the retina as a parallel rays, forming a full uniform circle of light **11**.
   Any obstacle or opacity **12** in the transparent parts of the eye that blocks the rays passage appears as a shadow **14** in this light circle.
**Fig 2b**: When the light source is located at a distance shorter than the eye's focal length **13**, the rays coming out of the lens diverge and a larger and somewhat less illuminated circle is viewed. Any opacity will appear enlarged.
**Fig 2c**: When the light source is located at a distance longer than the eye's focal length, the rays coming out of the lens converge and a smaller and brighter circle will be seen. Any opacity will appear smaller.

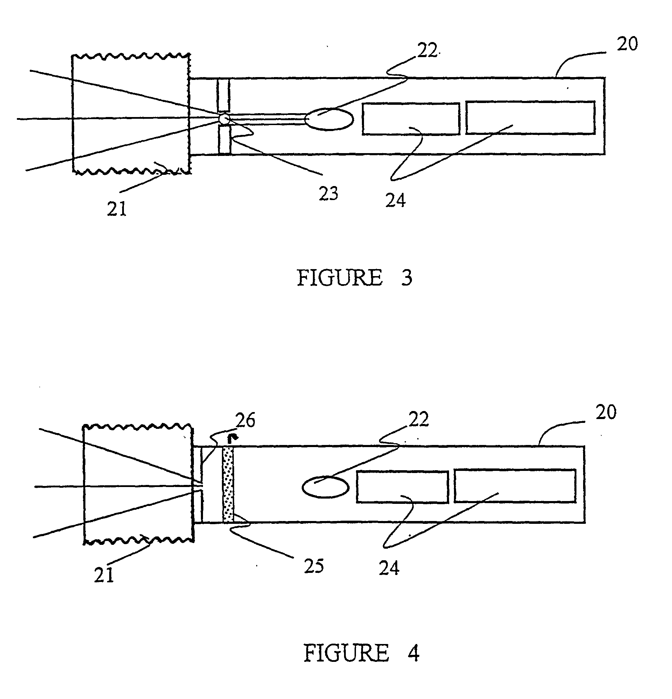

**Fig 3**: A preferred embodiment of a device according to the present invention. The device comprises a cylinder (20) closed on one side and having an eyeguard (21) on the open (or sealed with transparent window) front face. Inside the cylinder, aligned along the central axis are an incandescent light bulb (22) and a small glass bead (23). The image of the filament of the light bulb created by the glass bead being the (virtual) light source. The device further comprises of batteries (24) to supply the power for the light bulb.
**Fig 4:** A preferred embodiment of a device according to the present invention. The device comprises a cylinder (20) closed on one side and having an eyeguard (21) on the open (or sealed with transparent window) front face. Inside the cylinder, aligned along the central axis are an incandescent light bulb (22), a diffusing glass bead (25) and a pinhole disk (26), the pinhole being the (virtual) light source. The diffusing glass can be rotated thus averaging disturbances created by speckles in the light source or dust and dirt in the device. The pinhole is the (virtual) light source. The device further comprises of batteries (24) to supply the power for the light bulb.

### DETAILED DESCRIPTION OF THE INVENTION

The optical system of the eye (Fig 1), whose function is to bend rays of light which enter the eye so that an image of the view is projected on the retina (3), is composed of the cornea (1) and the lens (2). This multi-element lens (i.e. cornea + lens) is approximately equivalent to a thin lens with a typical average focal length of 16.67 mm which is located (1.5 mm behind the cornea) between the cornea and the lens. The plane at which this virtual equivalent lens is located is called the first principal plane of the eye (7).

When a small and uniformly diverging source of light is located at the principle focus of a convex lens, the light rays originating from the source and hitting the lens are bent at the lens and come out of the second surface of the lens as parallel rays (perpendicular to lens plane). In the same way, light rays emitted from a small diverging light source located in front of an eye, at a distance *d* equal to the focal length of the eye, fall on the retina in parallel lines. Thus, the light source will be seen by the viewer as a clear and uniformly illuminated circle (whose diameter is determined by the diameter of the pupil) provided there are no obstacles between the eye external surface and the retina. Any obstacles in the transparent parts of the eye that might block the rays passage will appear as shadows or spots in this light circle (Fig 2). These shadows are not necessarily darker than the background, they can sometimes appear as brighter spots, depending on the opacity refractive index with respect to the refractive index of the surrounding.

When the light source is located exactly at a distance *d* equal to the examined eye's focal length in front of the eye, a full circle of light (whose diameter is determined by the pupil diameter) falls on the retina and the viewer (i.e., the person to whom said eye belongs) sees a full illuminated circle.

When the light source is located at a distance shorter than the eye's focal length, the optical system of the eye cannot bend the rays enough to fully parallel rays and they are coming out of the second surface of the lens with diverging angle. Thus a larger and somewhat less illuminated circle is viewed.

When the light source is located at a distance longer than the eye's focal length, the rays coming from the lens fall on the retina with converging angle, thus a smaller and somewhat brighter circle is viewed.

Consequently, opacities in any part of the transparent optical system of the eye appear most clear as defined shadows when the light source is located at a distance which is approximately equal to the focal length. As the distance increases the shadows becomes smaller until they become too small to be observed. As the distance decreases the shadows become bigger but diffusive until the contrast between light and dark is difficult to define.

The device of the present invention is based on this phenomenon and exploits it for self diagnosis of opacities in the transparent optical system of the eye.

The device according to the present invention comprises a point (small) source of light enclosed inside a rigid frame wherein the rigid frame has means for fitting the frame to the eye orbit and wherein the light source is located within the frame such that when the frame is fitted to the eye the distance between the light source and the cornea is in the range of 5 to 30 mm

The rigid frame in which said light source is contained can be of any shape provided that when held correctly the distance of said light source from the eye is in the range of a focal length of an average eye.

The most commonly used means for positioning the device before the eye is a rubber eye guard as used in telescopes etc.

Since there are variations in the focal length of an eye between individuals the device according to the present invention can be designed such as to allow adjustment of the individual eye under examination.

Finer adjustment of the distance, in order to bring the center of the light source to the focal length of the examined eye, is done either by pressing an elastic eye guard against the eye orbit or in a device which has means for adjusting the position of the light source within the rigid frame, by using these means. These adjusting means can be in the form of a trail in the rigid frame and a screw that pushes the light source along this trail. Another arrangement of the device which allows adjustment of the light source position is telescopic-like body, i.e., an internal cylinder which can slide inside an external cylinder.

The light source can be a primary light source or a virtual light source, providing that the rays emitted from it diverge uniformly toward the eye (i.e., as if originating from the center of a sphere), such that when it is located at the focus of a convex lens, the rays coming out of the lens form a parallel and uniform cylinder of light (neither converging nor diverging).

In the context of the present invention such a light source is defined as a "small diverging source of light".

In the context of the present invention, the term "a primary light source" means the conventional meaning of the term "source of light" such as a lamp, i.e., where the rays of light originate from said source. The term "a virtual light source" means an element (or combination of elements) that directs light rays (originating from another "primary" source) from a small spot uniformly to all direction as if it was the origin of the light.

A primary source of light can be selected from light emitting diode (LED), diode laser or the edge of an elongated optic fiber.

A virtual source of light can be in the form of a pinhole disk or a glass bead or a polished metal sphere, illuminated by light that originates from another source of light which is either enclosed within the rigid frame or is external to the device.

The device can further comprise means for supplying power to the source of light.

The device according to the present invention can further comprise means for adjusting and controlling the intensity of the light, either by controlling the intensity of the light source by a rheostat or a dimmer or by a filter. In devices where the source of light is external to the device and where the device has a pinhole disk, the pinhole can optionally be of variable diameter allowing by this way controlling of the intensity of light that falls on the retina.

The device according to the present invention can further comprise a filter in order to select a specific range of the visible spectrum. For example, such a filter can select the "red" portion of the light to which the iris is less sensitive, thus allowing for a bigger circle of light to pass through the pupil and fall on the retina.

It can also comprise means to eliminate spots caused by the device itself and speckles of the light source by incorporating a spatial filter or by fast movement (preferably rotation) of the illuminating beam or of an element in the optical path of the beam.

In a preferred embodiment of the present invention (Fig 3), the rigid frame is a cylinder closed on one face and having an eyeguard on its second front open face wherein inside the cylinder, along its central axis there is a small incandescent bulb and said bulb illuminates a small glass bead located at the center of an opaque disk, and the device further includes batteries to power said bulb.

In another embodiment of the present invention, the rigid support is a closed cylinder with one transparent window and the source of light is a LED, or a diode laser or the edge of an elongated optical fiber aligned to a source of light.

Yet in another preferred embodiment of the present invention, one face of the rigid frame is open letting an external light to enter the device and passing through a glass bead or through a small pinhole located in the middle of an opaque partition.

## Claims

1. A device for self examination of the transparent optical system of the eye comprising; a) a rigid hollow frame having one face with means for fitting said frame to the orbit of the eye; b) a small source of light (10) located inside said frame ; c) means for adjusting the distance between the small source of light and cornea (2) such that when said frame is fitted to eye, said distance is in the range 5 to 30 mm, preferably 12 to 18 mm, and said device is **characterized by** further comprising means for clearing a beam of light from disturbances created by dust and dirt on the optical elements of the device or in the light passage to the eye or by speckles of the light source itself, said means are means for a fast rotation of the light source or of an element in the path of the light beam so that the disturbances are averaged out by the examined eye, said means for a fast rotation are either motorized or manual.

2. A device according to claim 1 wherein the distance between the source of light and cornea (2), when said frame is fitted to eye orbit, is approximately 15 mm.

3. A device according to claim 1 wherein the small source of light (10) is a primary source of light or a virtual source of light transmitting, reflecting or refracting light rays that originate from another light source.

4. A device according to claim 3 wherein the small source of light is a primary small source of light selected from light emitting diode (LED), diode laser or the edge of an elongated optic fiber.

5. A device according to claim 3 wherein the small source of light is a virtual source of light, in the form of a pinhole disk or a glass bead (23) or a polished metal sphere, illuminated by light that originates from another source of light.

6. A device according to claim 5 wherein the another source of light is enclosed within the rigid frame.

7. A device according to claims 4 or 6 wherein the device further comprising means for adjusting and controlling the intensity of the light.

8. A device according to claim 5 wherein the another source of light is external to the device.

9. A device according to claim 1 further comprising means for supplying power to the source of light.

10. A device according to claim 1 wherein the means for fitting the frame to the orbit of the eye is an elastic eye-guard (21).

11. A device according to claim 10 wherein the elastic eye-guard (21) also serves as the means for adjusting the distance between the light source and the cornea by pressing said elastic eye-guard against the eye orbit.

12. A device according to claim 1 further comprising means for adjusting the position of the small source of light within the rigid frame.

13. A device according to claim 12 wherein the means for adjusting the position of the light source within the rigid frame are selected from a trail in the rigid frame and a screw that pushes the light source along this trail or an internal cylinder which can slide inside an external cylinder.

14. A device according to claim 1 wherein said means are a spatial filter comprising of a pinhole disk (26) or a combination of a lens or a diffusing glass and a pinhole disk (26).

15. A device according to any of the preceding claim further comprising a filter for selecting a specific range of the visible spectrum.

16. A method for self examination of opacities within the transparent optical system of the eye comprising a) aligning a small source of light emitting diverging rays toward the eye, in front of the eye at a distance of about 15 mm from the comea; b) fine adjusting of the distance between the light source and the eye until a clear bright circle is observed by the examined eye; c) examining said circle for shadows wherein any shadow observed in the circle might indicate an opacity in the optical system of the eye,
and said method is **characterized by** further comprising the step of rotating or moving the source of light or an element in the optical path in order to distinguish between shadows resulting from opacities within the eye to shadows resulting from any dust and dirt obstacles on the light beam passage from the source of light to the eye or from speckles of the light source itself.

17. A method according to claim 16 wherein the source of light is a primary light source or a virtual light source transmitting, reflecting or refracting light from another source.

18. A method for self examination of opacities within the transparent optical system of the eye according to claim 16 by using a device as defined in claims 1 - 15.

## Patentansprüche

1. Vorrichtung zur Selbstuntersuchung des transparenten optischen Systems des Auges, mit: a) einem steifen hohlen Rahmen, der eine Fläche mit einer Einrichtung aufweist zum Aufsetzen des Rahmens an der Augenhohle; b) einer kleinen Lichtquelle (10), die innerhalb des Rahmens angeordnet ist; c) einer Einrichtung zur Einstellung des Abstands zwischen der kleinen Lichtquelle und der Augenhornhaut (2), derart, dass, wenn sich der Rahmen am Auge angeordnet befindet, der Abstand im Bereich von 5 bis 30 mm, vorzugsweise 12 bis 18 mm beträgt,
**dadurch gekennzeichnet, dass**
die Vorrichtung eine Einrichtung besitzt zur Beseitung von Störungen eines Lichtstrahls, die von Staub und Schmutz an den optischen Elementen der Vorrichtung oder auf dem Weg des Lichts zum Auge oder durch Flecken der Lichtquelle selbst entstehen, wobei die Einrichtung eine Einrichtung ist für eine schnelle Drehung der Lichtquelle oder eines Elements in dem Lichtstrahlengang, so dass die Störungen von dem untersuchten Auge ausgemittelt sind und die Mittel für eine schnelle Drehung entweder von einem Motor oder manuell betätigt sind.

2. Vorrichtung nach Anspruch 1, wobei der Abstand zwischen der Lichtquelle und der Augenhornhaut (2) etwa 15 mm beträgt, wenn der Rahmen an der Augenhöhle aufsitzt.

3. Vorrichtung nach Anspruch 1, wobei die kleine Lichtquelle (10) eine primäre Lichtquelle oder eine virtuelle Lichtquelle ist, die Lichtstrahlen, die von einer anderen Lichtquelle ausgehen, überträgt, reflektiert oder bricht.

4. Vorrichtung nach Anspruch 3, wobei die kleine Lichtquelle eine primäre kleine Lichtquelle ist, ausgewählt aus der Gruppe einer lichtaussendenden Diode (LED), einer Laserdiode oder dem Rand einer langgestreckten optischen Faser.

5. Vorrichtung nach Anspruch 3, wobei die kleine Lichtquelle eine virtuelle Lichtquelle ist in der Form einer Pinhole-Scheibe oder einer Glasperle (23) oder einer polierten Metallkugel, die beleuchtet ist von Licht, das von einer anderen Lichtquelle stammt.

6. Vorrichtung nach Anspruch 5, wobei die andere Lichtquelle innerhalb des steifen Rahmens eingeschlossen ist.

7. Vorrichtung nach Anspruch 4 oder 6, wobei die Vorrichtung weiterhin eine Einrichtung zum Einstellen und Steuern der Intensität des Lichts aufweist.

8. Vorrichtung nach Anspruch 5, wobei die andere Lichtquelle außerhalb der Vorrichtung liegt.

9. Vorrichtung nach Anspruch 1, weiterhin **gekennzeichnet durch** eine Einrichtung zur Zufuhr von Energie an die Lichtquelle.

10. Vorrichtung nach Anspruch 1, wobei die Vorrichtung zur Anordnung des Rahmens an der Augenhöhle ein elastischer Augenschutz (21) ist .

11. Vorrichtung nach Anspruch 10, wobei der elastische Augenschutz (21) auch als Einrichtung dient zur Einstellung des Abstands zwischen der Lichtquelle und der Augenhornhaut durch Andrücken des elastischen Augenschutzes gegen die Augenhöhle.

12. Vorrichtung nach Anspruch 1, weiterhin **gekennzeichnet durch** eine Einrichtung **zur** Einstellung der Position der kleinen Lichtquelle innerhalb des steifen Rahmens.

13. Vorrichtung nach Anspruch 12, wobei die Einrichtung zur Einstellung der Position der Lichtquelle innerhalb des steifen Rahmens ausgewählt ist aus einer Führung in dem steifen Rahmen und einer Schraube, die die Lichtquelle entlang dieser Führung beaufschlägt oder einem innenliegenden Zylinder, der innerhalb eines außenliegenden Zylinders verschiebbar ist.

14. Vorrichtung nach Anspruch 1, wobei die Einrichtung ein räumlicher Filter ist mit einer Pinhole-Scheibe (26) oder eine Kombination aus einer Linse oder einem Diffusionsglas und einer Pinhole-Scheibe (26).

15. Vorrichtung nach einem der vorstehenden Ansprüche, weiterhin **gekennzeichnet durch** einen Filter zur Auswahl eines spezifischen Bereichs des sichtbaren Spektrums.

16. Verfahren zur Selbstuntersuchung von Trübungen innerhalb des transparenten optischen Systems des Auges, **gekennzeichnet durch** die Schritte a) Ausrichten einer kleinen Lichtquelle, die sich ausbreitende Strahlen zu dem Auge aussendet, vor dem Auge in einem Abstand von etwa 15 mm von der Augenhornhaut; b) Feineinstellung des Abstands zwischen der Lichtquelle und dem Auge, bis ein deutlicher heller Kreis von dem untersuchten Auge beobachtet ist; c) Untersuchen des Kreises auf Schatten hin, wodurch jeder in dem Kreis beobachtete Schatten auf eine Trübung in dem optischen System des Auges hinweisen könnte, **gekennzeichnet durch** die Schritte der Drehung oder. Bewegung der Lichtquelle oder eines Elements auf dem optischen Weg zur Unterscheidung zwischen Schatten, die von Trübungen innerhalb des Auges stammen und Schatten, die von Staub und Schmutzteilchen auf dem Lichtstrahlengang von der Lichtquelle zum Auge oder von Flecken an der Lichtquelle selbst stammen.

17. Verfahren nach Anspruch 16, wobei die Lichtquelle eine primäre Lichtquelle oder eine virtuelle Lichtquelle ist, die Licht von einer anderen Quelle überträgt, reflektiert oder bricht.

18. Verfahren zur Selbstuntersuchung von Trübungen innerhalb des transparenten optischen Systems des Auges nach Anspruch 16 unter Verwendung einer Vorrichtung nach Anspruch 1 bis 15.

## Revendications

1. Dispositif d'auto-examen du système optique transparent de l'oeil comprenant :
a) un cadre creux rigide présentant une face avec moyens pour adapter le cadre à l' orbite de l'oeil ;
b) une petit source lumineuse (10) placée à l'intérieur dudit cadre ;
c) des moyens de réglage de la distance entre la petite source lumineuse et la cornée (2) de sorte que, lorsque ledit cadre est adapté à l'oeil, ladite distance est dans la plage de 5 à 30 mm, de préférence de 12 à 18 mm,
ledit dispositif étant **caractérisé en ce qu'**il comprend en outre des moyens permettant de débarrasser un faisceau lumineux des parasites générés par des poussières et des salissures présents sur les éléments optiques du dispositif ou bien sur le trajet de la lumière vers l'oeil ou encore par des taches provenant de la source lumineuse elle-même, lesdits moyens étant des moyens de mise rotation rapide de la source lumineuse ou d'un élément sur le trajet du faisceau lumineux de sorte que l'oeil examiné fait la moyenne de ces parasites, lesdits moyens de mise en rotation rapide étant des moyens motorisés ou manuels.

2. Dispositif selon la revendication 1, dans lequel la distance entre la source lumineuse et la cornée (2), lorsque ledit cadre est adapté à l'orbite oculaire, est approximativement de 15 mm.

3. Dispositif selon la revendication 1, dans lequel la petite source lumineuse (10) est une source lumineuse primaire ou une source lumineuse virtuelle transmettant, réfléchissant ou réfractant des rayons lumineux qui proviennent d'une autre source lumineuse.

4. Dispositif selon la revendication 3, dans lequel la petite source lumineuse est une petite source lumineuse primaire choisie parmi une diode électroluminescente (LED), un laser à diode ou le bord d'une longue fibre optique.

5. Dispositif selon la revendication 3, dans lequel la petite source lumineuse est une source lumineuse virtuelle, se présentant sous la forme d'un disque à trou d'épingle ou bien d'une bille de verre (23) ou encore d'une sphère métallique polie, éclairée par la lumière qui provient d'une autre source lumineuse.

6. Dispositif selon la revendication 5, dans lequel l'autre source lumineuse est enfermée à l'intérieur du cadre rigide.

7. Dispositif selon les revendications 4 ou 6, dans lequel le dispositif comprend en outre des moyens de réglage et de contrôle de l'intensité lumineuse.

8. Dispositif selon la revendication 5, dans lequel l'autre source lumineuse est extérieure au dispositif.

9. Dispositif selon la revendication 1, comprenant en outre des moyens d'alimentation en énergie de la source lumineuse.

10. Dispositif selon la revendication 1, dans lequel les moyens pour adapter le cadre à l'orbite oculaire sont un protège-oeil élastique (21).

11. Dispositif selon la revendication 10, dans lequel le protège-oeil élastique (21) sert également de moyens de réglage de la distance entre la source lumineuse et la cornée en pressant ledit protège-oeil élastique contre l'orbite oculaire.

12. Dispositif selon la revendication 1, comprenant en outre des moyens de réglage de la position de la petite source lumineuse à l'intérieur du cadre rigide.

13. Dispositif selon la revendication 12, dans lequel les moyens de réglage de la position de la source lumineuse à l'intérieur du cadre rigide sont sélectionnés parmi une piste dans le cadre rigide et une vis qui pousse la source lumineuse le long de cette piste ou bien un cylindre interne qui peut coulisser à l'intérieur d'un cylindre externe.

14. Dispositif selon la revendication 1, dans lequel lesdits moyens sont un filtre spatial comprenant un disque à trou d'épingle (26) ou une combinaison d'une lentille ou d'un verre diffusant et d'un disque à trou d'épingle (26).

15. Dispositif selon l'une quelconque des revendications précédentes, comprenant en outre un filtre pour sélectionner une plage spécifique du spectre visible.

16. Procédé d'auto-examen des opacités à l'intérieur du système optique transparent de l'oeil comprenant
a) l'alignement d'une petite source lumineuse émettant des rayons divergents en direction de l'oeil, en face de l'oeil à une distance d'environ 15 mm de la cornée ;
b) le réglage fin de la distance entre la source lumineuse et l'oeil jusqu' à ce que l'oeil examiné observe un cercle clair et brillant ;
c) l'examen d'ombre dans ledit cercle, toute ombre observée dans le cercle pouvant indiquer une opacité dans le système optique de l'oeil,
ledit procédé étant **caractérisé en ce qu'**il comprend en outre l'étape consistant à faire tourner ou à déplacer la source lumineuse ou un élément sur le trajet optique afin de distinguer les ombres résultant des opacités à l'intérieur de l'oeil des ombres résultant d'obstacles se présentant sous la forme de poussières et de salissures sur le trajet du faisceau lumineux entre la source lumineuse et l'oeil ou résultant de taches provenant de la source lumineuse elle-même.

17. Procédé selon la revendication 16, dans lequel la source lumineuse est une source lumineuse primaire ou une source lumineuse virtuelle transmettant, réfléchissant ou réfractant la lumière provenant d'une autre source.

18. Procédé d'auto-examen des opacités à l'intérieur du système optique transparent de l'oeil selon la revendication 16 en utilisant un dispositif tel que défini dans les revendications 1 à 15.
